# EUROPEAN PATENT SPECIFICATION

(11) **EP 3 184 128 B1**
(45) Date of publication and mention of the grant of the patent: **11.12.2019**
(21) Application number: 15201547.5
(22) Date of filing: 21.12.2015
(51) Int. Cl.: A61L 27/36, A61L 27/38

(54) **METHOD FOR PRODUCING A SUPPORT DEVICE**
VERFAHREN ZUR HERSTELLUNG EINER TRÄGERVORRICHTUNG
PROCÉDÉ DE FABRICATION D'UN DISPOSITIF DE SUPPORT

(43) Date of publication of application: 28.06.2017
(73) Proprietor: Heinrich Heine Universität Düsseldorf, 40225 Düsseldorf (DE)
(72) Inventor: Aubin, Hug, 40219 Düsseldorf (DE); Lichtenberg, Artur, 40629 Düsseldorf (DE); Akhyari, Payam, 40225 Düsseldorf (DE)
(74) Representative: Remus, Alvaro Johannes

(56) References cited:
- EP-A1- 2 842 581
- WO-A1-2007/025233

## Description

### Background of the invention

The invention relates to a method for producing a support device, wherein the method comprises providing a whole heart comprising an extracellular matrix and cells attached thereto and removing all cells from the extracellular matrix so as to produce a decellularized extracellular matrix scaffold, wherein the extracellular matrix scaffold is processed so as to produce a sleeve-like support device which is designed to circumferentially encase a living heart. The invention further concerns a support device comprising a decellularized extracellular matrix scaffold of a heart, said extracellular matrix scaffold being designed like a sleeve to circumferentially encase a living heart.

Rising epidemiologic numbers of cardiovascular disease and the current demographic trend turn heart disease into an increasing health-economic problem worldwide. The causal therapy of end-stage heart disease is possible only in a small number of selected patients. In the vast majority of patients though, the etiology of heart disease is multifactorial making it impossible to eliminate all causal factors completely. The current symptomatic treatment of end-stage heart failure is classically based on three pillars, the general-non-pharmacological, the pharmacological and the interventional or surgical therapy. However, those measures can only help to slow the progression of the disease but cannot prevent the degeneration and pathologic remodeling of the diseased heart muscle, which explains the existing high morbidity and mortality of heart disease which will even further increase in the future with the current demographic trend.

Heart transplantation is currently the only curative treatment of end-stage heart failure because of the lack of sufficient myocardial regeneration. However, the worldwide shortage of donor organs with a striking discrepancy between patients on the waiting lists and patients that actually profit from a heart transplantation obliges us to seek for alternative therapies that will benefit all patients.

The rising numbers of cardiovascular disease and donor organ shortage increase the need for alternative therapies of end-stage heart failure. Although the pharmacological treatment of heart disease has shown major improvements in the last decades increasing quality of life and survival rates it still remains an almost purely symptomatic treatment. Progression of the disease and degeneration of the injured myocardial tissue can be slowed down but not stopped let alone reversed by pharmacological treatment alone.

### Prior art

Surgical therapies other than the heart transplantation and improved implantable cardioverter-defibrillators and cardiac assist devices have remained of experimental character for a while now. Promising approaches as for example the active cardiomyoplasty, in which the *latissimus dorsi* muscle is wrapped around the heart to support contraction, have not proven to be viable in the clinical praxis because of the high complexity of the procedure and poor results (Gummert et al., 2004). Nonetheless, in the framework of dynamic cardiomyoplasty studies it has been shown that the passive support of the damaged heart with the non-stimulated and fibrosed *latissimus* muscle improved hemodynamics. This led to the development of a variety of synthetic materials for the so-called passive cardiomyoplasty, which improves heart function and slows progression of the disease by preventing left ventricular dilatation (Grothues et al., 2006; Mann et al., 2011). By today the passive cardiomyoplasty has become a well-studied surgical procedure in the treatment of heart failure, however, the supposed benefit of the procedure still not outweighs its risks in most cases being the reason why it has not been able to revolutionize surgical treatment of heart disease yet, not even being part of the clinical routine by now.

US 7 404 793 B2 discloses a method and apparatus for treating heart failure, wherein a harness made of a synthetic shape memory material is configured to be placed about at least a portion of a patient's heart to apply a mild compressive force on the heart over a range of elastic deformation of the harness. This harness can be shifted to a second range of deformation, wherein it is shifted to this second range of deformation by application of a stimulus or alteration of environmental conditions beyond a threshold level.

US 2010/0167373 A1 discloses a pouch-like construct comprising a scaffold substance such as collagen type I and mammalian cells, which can be used for preventing distension and/or resisting dilation of the heart in a mammal. The pouch-like construct has contractile properties and a bag-shaped structure with an opening. The construct is shaped in such a manner that it is suitable for enclosing and compressing at least part of the heart of a mammal. The pouch-like construct comprises or consists of mammalian tissue comprising cardiac myocytes. In general, the pouch-like constructs can be generated from muscle and non-muscle cells as well as from mixtures of muscle and non-muscle cells.

EP 1 928 519 B1 discloses methods and materials to decellularize an organ or tissue as well as methods and materials to recellularize a decellularized organ or tissue. In particular, the invention provides for a decellularized mammalian heart, including a decellularized extracellular matrix of the heart that has an exterior surface. The extracellular matrix of the decellularized heart substantially retains the morphology of the extracellular matrix prior to decellularization, and the exterior surface of the extracellular matrix is substantially intact.

Since the inventors of the whole-heart decellularization concept disclosed in EP 1 928 519 B1 provided this method, it is possible to generate native extracellular matrix (ECM) derived 3D scaffolds that preserve the basic anatomy, vasculature network and basic ECM characteristics of the native heart (Mann et al., 2011). However, using a whole-heart scaffold to create a one-to-one substitute for a diseased heart still poses an insuperable challenge in the near future because of the tremendous complexity of the native heart.

EP 2 842 581 A1 discloses a pouch-like structure useful for mechanically preventing distension and/or resisting dilation of the heart and for supporting the heart's function by controllable and paracrine support of a failing heart in a mammal. The pouch-like structure is composed at least partly of engineered tissue comprising genetically engineered cells, like, genetically engineered cells other than cardiac myocytes, which contain a gene encoding a paracrine factor, said gene encoding the paracrine factor being under control of an inducible promoter system or an heterologous promoter system. The pouch-like structure is based on engineered tissue obtained from cells cultured in a scaffold.

### Summary of the invention

It is the objective of the invention to provide a biocompatible device for promoting regeneration of the dysfunctional myocardium and improving function of a diseased heart, and a method for producing such device.

The scope of this invention is defined by the claims. Any references in the description to methods of treatment refer to the devices of the present invention for use in a method for treatment of the human (or animal) body by therapy.

The objective is addressed by a method for producing a support device as initially specified, wherein the extracellular matrix scaffold is processed by removing at least one part of at least one of the heart structures selected from the group consisting of the heart base, the apex, and the septal wall. At first, all cells are removed from the extracellular matrix of a whole heart according to the methods known in the art. Hereby a decellularized extracellular matrix (ECM) scaffold directly derived from native cardiac tissue is produced, which is then processed in order to generate a kind of myocardial, sleeve-like device that can provide structural support to an injured ventricle so as to prevent progressive ventricular dilatation and thus improve diastolic function. Processing is accomplished such that the decellularized ECM scaffold is tailored to fit onto a living heart so that the heart is circumferentially encased by the scaffold. If such tailored ECM scaffold is imposed on the injured heart, it stabilizes the ventricles and supports their diastolic function. Such biocompatible ECM scaffold has similar architecture and biomechanical properties as the native myocardium and thus additionally induces physiological scaffold and native tissue interaction. This leads to, e.g., *in vivo* recruitment of regenerative cells or induction of angiogenesis in ischemic myocardial areas enhancing myocardial regeneration, such as a kind of bioactive cardiomyoplasty. The produced support device provides host and possible donor cells with a biomimetic scaffold that is directly derived from their natural environment mimicking its unique characteristics to an unmatched degree with minimal immunogenic response. Accordingly, the method according to the invention results in a beneficial device that represents a promising, alternative therapeutic tool for end-stage heart failure for clinical use in humans.

To this end, the invention provides a method for producing a support device, said method comprising:
a) providing a whole heart comprising an extracellular matrix and cells attached thereto;
b) removing all cells from the extracellular matrix so as to produce a decellularized extracellular matrix scaffold; and
c) processing the extracellular matrix scaffold so as to produce a sleeve-like support device which is designed to circumferentially encase a living heart, wherein the extracellular matrix scaffold is processed by removing at least one part of at least one of the heart structures selected from the group consisting of the heart base, the apex, and the septal wall.

According to the invention the extracellular matrix scaffold is processed in step c) by removing at least one part of at least one of the heart structures selected from the group consisting of the heart base, the apex, and the septal wall. Accordingly, the ECM scaffold can be tailored by removing one or more of these heart structures so as to shape the scaffold as a sleeve-like jacket or mantle that circumferentially encases the injured heart. To this end, all heart structures that could interfere with fitting the ECM scaffold onto the surface of the heart have to be removed, for example, by dissecting the scaffold with an appropriate tool. On the other hand, if processing is limited to the removal of the hindering heart structures, the main characteristics of the extracellular matrix as well as the geometry and the vascular system of the native heart can be maintained.

In an exemplary advantageous embodiment of the invention the extracellular matrix scaffold is processed in step c) by treating the surface of the extracellular matrix scaffold. The surface of the EMC scaffold can be treated to adapt the scaffold to specific applications and/or to provide it with useful properties that further improve its bioactive activities. Such treatment can be performed, for example, by laser manipulation techniques.

In a further exemplary advantageous embodiment of the invention the endocardial layer on at least a part of the surface of the extracellular matrix scaffold is at least in part destroyed and/or removed. By partly destroying or removing the endocardial layer, the surface of the ECM scaffold is structured to enable enhanced host and/or donor cell penetration into the scaffold.

In addition to processing, the extracellular matrix scaffold can be customized in order to adapt it to specific applications. Customizing may, for example, include functionalization of the ECM surface in order to provide it with other and/or additional properties. For example, the ECM scaffold can be specifically adapted to the host pathology, e.g., ischemic myocardial injury or structural disarray of myocardial fibers as in dilatative cardiomyopathy. Moreover, physiological scaffold and native tissue interaction can be directed by customization.

In an exemplary advantageous embodiment of the invention the extracellular matrix scaffold is customized by coating the surface of the extracellular matrix scaffold. According to the invention the inner or the outer surface, or both, of the ECM scaffold can be coated. For example, the surface of the extracellular matrix scaffold may be coated with at least one growth factor, aptamer, RGD peptide, pharmacologically active substance, and/or biodegradable material.

In another exemplary advantageous embodiment of the invention the extracellular matrix scaffold is customized by recolonizing the extracellular matrix with living cells. For example, the extracellular matrix scaffold may be recolonized with at least one cell selected from the group consisting of tissue derived progenitor or stem cells, circulating endothelial progenitor cells, autologous or allogene stem cells, and bone marrow derived mesenchyme stem cells.

The objective is further addressed by a support device comprising a decellularized extracellular matrix scaffold of a heart as initially specified, wherein the extracellular matrix scaffold is devoid of at least one part of at least one of the heart structures selected from the group consisting of the heart base, the apex, and the septal wall. That is, the invention provides a kind of tissue engineered myocardial sleeve (TEMS) based on a bioactive extracellular matrix (ECM) scaffold directly derived from native cardiac tissue in form of a decellularized whole-heart scaffold. This support device provides structural support to injured ventricles preventing progressive ventricular dilatation and improves the function of the diseased heart by improving its diastolic function. Moreover, as the support device is a customizable bioactive scaffold having similar architecture and biomechanical properties as the native myocardium, it promotes regeneration of the dysfunctional myocardium and additionally induces physiological scaffold and native tissue interaction. Such interaction leads to *in vivo* recruitment of regenerative cells or induction of angiogenesis in ischemic myocardial areas, so that myocardial regeneration is enhanced. The support device according to the invention provides host and possible donor cells a biomimetic scaffold that is directly derived from their natural environment mimicking its unique characteristics to an unmatched degree with minimal immunogenic response. The support device according to the invention is a promising, alternative therapeutic tool for end-stage heart failure for clinical use in humans.

In an exemplary advantageous embodiment of the invention the extracellular matrix scaffold comprises the free walls of the left and right ventricles of the heart. Accordingly, the device according to the invention is designed as a sleeve-like jacket or mantle that can be used to circumferentially encase the injured heart, especially its ventricles. Advantageously, the device is devoid of any heart structure that could interfere with fitting it onto the surface of a living heart, while the main characteristics of the extracellular matrix as well as the geometry and the vascular system of the native heart are present.

In order to enable enhanced host and/or donor cell penetration into the extracellular matrix scaffold, the device according to the invention can be designed such that at least a part of the surface of the ECM scaffold is devoid of an endocardial layer.

The extracellular matrix scaffold of the support device according to the invention may be coated, for example, with at least one growth factor, aptamer, RGD peptide, pharmacologically active substance, and/or biodegradable material.

In an exemplary advantageous embodiment of the invention the extracellular matrix scaffold of the support device according to the invention is colonized with living cells. For example, the extracellular matrix scaffold may be colonized with at least one cell selected from the group consisting of tissue derived progenitor or stem cells, circulating endothelial progenitor cells, autologous or allogene stem cells, and bone marrow derived mesenchyme stem cells.

### Description of exemplary and advantageous embodiments of the invention

The method according to the invention may comprise the following steps:
a) Providing a whole-heart from a (human) donor;
b) *In toto* decellularization of the heart; and
c) Processing of the acellular whole-heart extracellular matrix (ECM) scaffold into a sleeve-like device (tissue engineered myocardial sleeve = TEMS);
d) Optionally, biological customization of the device.

The device produced according to the inventive method can be surgically fitted onto the living heart of a (human) host by imposing it on the organ manually. The device implantation into the (human) host can be carried out by standard surgical procedures.

### In toto decellularization of a heart:

Donor hearts can be either of porcine or human cadaveric origin, and may be size-matched to the potential host. Donor hearts are decellularized through automated software-controlled retrograde aortic perfusion based on a customized organ perfusion system for whole-organ decellularization according to Akhyari et al. (2011), ensuring standardization in terms of decellularization efficacy and ECM integrity on a macro- as well as micromolecular level.

A fully automated software-operated control system and method for whole-heart decellularization through automated long-term retrograde aortic perfusion of the explanted rat hearts that allows for coronary perfusion with a standardized constant perfusion pressure was developed by Aubin et al. (Methods Mol Biol., 2013). In this method, the heart is inserted in a closed perfusion cycle consisting of a modified 100 ml glass flask, serving as perfusion chamber connected to a silicon tube system, with an additional volume of 20 ml. Coronary perfusion is enabled by retrograde aortic perfusion through connection of the thoracic aortic cannula to the tube system serving as perfusion inlet. During the decellularization process the explanted heart floats freely in the perfusate attached only at the perfusion inlet with the perfusate draining out of the heart trough the right atrium. The tube system is clamped into a peristaltic pump that drives the perfusate circulation. For automated software-operated perfusion, a data acquisition device is connected to a pressure transducer that is connected in series to the perfusion inlet via an analog input module and to the peristaltic pump via an analog output module. Over an operational computer connected to the data acquisition device, an ad hoc-designed software is employed as control software, allowing the determination of a pressure set point for the coronary perfusion and operating the peristaltic pump online driving perfusate circulation with the determined perfusion pressure. Through a closed-loop control via a PID (proportional-integral-derivative) control algorithm embedded into the control software, the preset perfusion pressure is automatically kept constant during the entire heart decellularization process through online adaption of the pump flow rate. This guarantees constant coronary perfusion pressure despite varying vascular tone along the process. For rat heart decellularization, a constant coronary perfusion between 75 and 80 mmHg has proven to be most efficient. Additionally, the control software visualizes all data online and records them as a .xlsx file for documentation and further analysis purposes. This allows to retrospectively exclude hearts from the experimental group before analysis, which most probably will show inefficient decellularization because of inconstant perfusion pressure, for example, because of aortic insufficiency or coronary burst.

### Decellularization Protocol:

A steadily growing number of different decellularization protocols can be found in the literature (Crapo at al., 2011). Although the decellularization processes vary widely, four major steps are classically needed for the decellularization of native tissues:
1. Lysis of the cell membranes;
2. Separation of the cellular components from the ECM;
3. Solubilization of cytoplasmatic and nuclear components; and
4. Removal of cell debris.

Commonly used decellularization agents and techniques are described by Crapo at al. (2011). The most usual decellularization agents for perfusion based decellularization include hypo- and hypertonic solutions as well as non-ionic (e.g. Triton X-100) and ionic detergents (e.g. DCA and SDS) in different combinations and varying concentrations.

A typical protocol for use in the decellularization of rodent hearts can be found in a publication of Aubin et al. (Methods Mol Biol., 2013). Although it has already been shown that perfusion based organ decellularization protocols are also feasible in larger animal models (e.g. porcine) with comparable results, this small animal protocol might have to be adapted for the larger donor hearts.

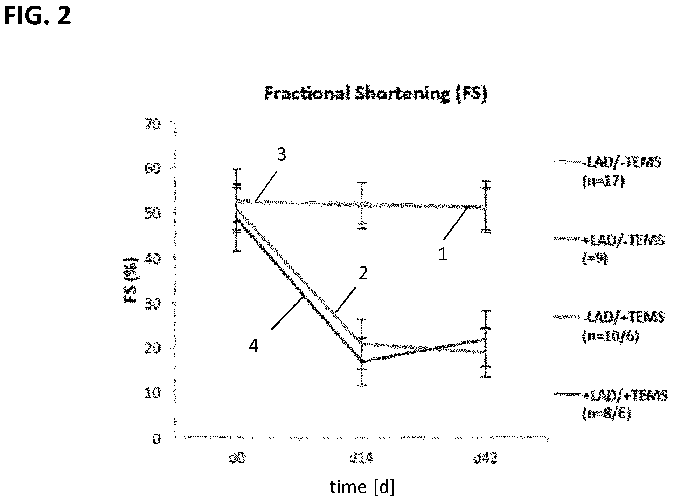

Decellularization protocol for rodent heart decellularization (Aubin et al., Methods Mol Biol., 2013):
1. Prefill the perfusion cycle with heparinized PBS (10 IU/ml Heparin) containing 10 mM adenosine.
2. Place the heart into the perfusion chamber by connecting the aortic cannula to the perfusion inlet. Close the perfusion chamber.
3. Start perfusate circulation with a coronary perfusion pressure of 77.5 mmHg manually or through the automatic software-operated control system. Perfusate is kept at room temperature unless otherwise stated.
4. Perfuse for 15 min with heparinized PBS (10 IU/ml Heparin) containing 10 mM adenosine (see Note 5).
5. Perfuse for 15 min with deionized water (see Note 6).
6. Perfuse for 12 h with 1 % SDS, 1 % DCA, and 0.05 % sodium azide in deionized water (see Notes 7-9).
7. Perfuse for 15 min with deionized water.
8. Perfuse for 12 h with 20 % glycerol, 0.05 % sodium azide, and 25 mM EDTA in 0.9 % NaCl solution.
9. Perfuse for 15 min with deionized water.
10. Perfuse for 12 h with 1 % saponin and 0.05 % sodium azide in deionized water.
11. Perfuse for 15 min with deionized water.
12. Perfuse for 12 h with 20 % glycerol, 0.05 % sodium azide, and 25 mM EDTA in 0.9 % NaCl solution.
13. Perfuse for 15 min with deionized water.
14. Perfuse for 12 h with 200 IU/ml DNase I and 50 mM MgCl in PBS at 37°C (see Note 10).
15. Perfuse for 12 h with 100 IU/ml penicillin-streptomycin in PBS (see Note 11).
16. Remove the decellularized heart from the perfusion chamber and store in PBS supplemented with 100 IU/ml penicillin-streptomycin at 4 °C (see Note 12) (see Fig. 2).

### Quality control after decellularization process:

After the decellularization process tissue samples from parts not needed for the TEMS (such as septum and apex of the heart) are analyzed for decellularization efficacy as a form of quality control and standardization accounting for the common variances in the processing of biological materials and in order to prevent adverse immunogenic reactions in cases of insufficient donor cell removal. Such quality control includes histological evaluation cellular remnants and ECM integrity by standard histological staining and analysis, and standard biomolecular analysis of DNA remnants and ECM components.

### Processing of acellular whole-heart scaffold into TEMS:

### Manual Dissection:

In order to generate the TEMS scaffold, the acellular whole-heart scaffold is manually dissected (this step could also possibly be automatized) by first removing the heart base at the subvalvular level, then the apex and finally the septal wall, remaining with a "sleeve-like" ECM-scaffold consisting of the free walls of the left and right ventricle with intact macro- and microscopic architecture.

### Laser-processing of the TEMS-tissue interface:

In order to enhance TEMS-tissue interaction after implantation, the TEMS-tissue interface which is formed by the former endocardial side of the left and right ventricle of the donor heart, is further processed with laser manipulation techniques. Goal of this processing step is to interrupt the endocardial layer, which remains intact after decellularization, without altering the underlying ECM. This step is critical to enable enhanced host and/or donor cell penetration into the TEMS-ECM.

### For example, the following strategies are possible:

- Complete abrasion of the endocardial layer of the whole TEMS;
- Selective abrasion of the endocardial layer only at the interface of TEMS and pathologic native tissue (only at the TEMS-tissue interface corresponding to the dysfunctional myocardial site);
- Creation of selective cell entry points (= pores of varying size and depth) with intact surrounding endocardial layer; and
- Creation of micropores through the whole TEMS (endocardial layer through epicardial layer and underlying ECM).

### Biological customization:

Biological functionalization of the TEMS-tissue interface allows to individually customize the TEMS (cTEMS) for specific applications. Hence, cTEMS may possibly even be specifically adapted to the host pathology, e.g., ischemic myocardial injury, or structural disarray of myocardial fibers as in dilatative cardiomyopathy. Some experience on the biological functionalization of decellularized tissues was described by Assmann at al. (2013).

Two different strategies for the biofunctionalization of the TEMS-tissue interface is feasible, either the direct coating of the TEMS inner surface with a biological active material or the coating of the TEMS inner surface with a biocompatible carrier substance (e.g. a variant of hydrogel, e.g. Hyaluronic acid based, GelMA) that could further be functionalized.

Assmann et al. (2013) have described surface coating of decellularized aortic conduits:
The covalent binding of the fluorochrome Alexa488 (Invitrogen, Carlsbad, USA) to fibronectin isolated from human blood plasma was conducted according to Huynh et al., unpublished data, 2013. In brief, fibronectin solution (1 mg/ml fibronectin in PBS, pH 7.3) was mixed with sodium bicarbonate at the final concentration of 0.1 M (pH 8.7) for amine labeling according to the user's manual. Labeling was done by adding 80-fold molar excess of Alexa488 to the fibronectin solution and incubating for 1 h in dark at room temperature with gentle rotation. Free dyes were removed by dialysis with PBS (pH 7.3) overnight. Concentrations and ratios of dye/fibronectin molecule conjugation were determined by reading the absorption at 280 nm, 496 nm and calculated according to the user's manual. Batches of Alexa488-labeled fibronectin with ratios between six and nine were chosen for further experiments. In order to reveal the optimal fibronectin coating conditions for decellularized rat aortic conduits, different concentrations of the fibronectin coating solution (20, 50, 100 and 200 mg/ml) as well as different coating conditions, including different temperatures (20 and 37 C) and static versus slightly agitated incubation, were tested prior to the experiments. With regard to the coating efficiency as well as to economic aspects, all decellularized rat aortic conduits intended for implantation (n ¼ 18) were incubated with covalently Alexa488-labeled fibronectin under the following conditions: fibronectin concentration 50 mg/ml in PBS, incubation time 24 h and incubation temperature 37 C. After resection of a distal ring of the conduit for decellularization quality assurance and for confirmation of the coating efficiency, the grafts were instantaneously implanted avoiding prolonged exposure to light to prevent bleaching of the fluorophore.

Surface-modified hyaluronic acid hydrogels to capture endothelial progenitor cells are described by Camci-Unal et al. (2010):

### Production of photocrosslinkable HA precursors (methacrylated HA):

1 gram of sodium hyaluronate was added to 100 mL of water. After obtaining a homogenous solution 1 mL of methacrylic anhydride was added, then the pH of the mixture was adjusted to 8- 9 and maintained in the same range throughout the reaction. The reaction was carried out on ice for 24 h. The final product was dialyzed against deionized water for 3 days at 4 °C and the resulting solution was then lyophilized for 3 days. 1H NMR analysis indicated a degree of methacrylation of 16%.

### Production of HA-based hydrogels:

1% (w/v) methacrylated HA was prepared in PBS solution containing 0.5% (w/v) photoinitiator. This prepolymer solution was kept at 80 °C until the HA completely dissolved. To enable spreading of EPCs to endothelialize the surface, hydrogels containing 1% (w/v) methacrylated HA, 2% (w/v) methacrylated gelatin and 0.5% (w/v) photoinitiator were also made. After complete dissolution of solid components prepolymer solutions were exposed to UV-light for 180 s at 5.5 mW cm°² between two untreated glass slides with 1 mm thick spacers. Following photopolymerization, the two glass slides were separated and 4 mm diameter hydrogel samples were punched using biopsy punches. A thin HA layer was coated on 3-(trimethoxysilyl) propyl methacrylate (TMSPMA) treated glass slides to attach the punched hydrogel samples. After this step antibody immobilization was accomplished.

### Confirmation of covalent attachment on HA hydrogels:

To conjugate CD34 antibody on the surface of gels, HA hydrogels were incubated in 3-(N-morpholino) propanesulfonic acid (MES) buffer (pH 5.6) for 30 min. After removing the buffer, the 0.1 M N-(3-dimethylaminopropyl)-N'-ethylcarbodiimide (EDC) and 0.2 M N-hydroxysuccinimide (NHS) mixture were added to activate the carboxylate groups on HA hydrogels. This mixture was incubated on a shaker for 1 h and then the EDC/NHS solution was removed. Activated hydrogels were washed in PBS for 10 min. Upon removal of PBS, 1, 10 or 25 µg·ml⁻¹ FITC conjugated or non-labeled anti-human CD34 antibody was added on the top surface of the hydrogels and the resulting material was incubated at 4 °C overnight. These hydrogels were then kept in PBS overnight to allow for desorption of nonspecifically adsorbed antibodies. All experiments were carried out in triplicate. Dimensionless fluorescent intensity values were obtained utilizing ImageJ analysis by dividing the fluorescence with the background value and plotted against antibody concentration.

Furthermore, both the TEMS-tissue interface itself and the above mentioned carrier substance may be cell seeded. The former may even be embedded with cells with regenerative and/or contractile potential ex vivo for targeted cell delivery, or functionalized with pharmacologically active substances for targeted drug delivery on the site of diseased myocardium.

### Possible biofunctionalization strategies are:

- Coating of the TEMS-tissue interface with biological active substances either directly or an a biocompatible carrier in order to promote targeted cell recruitment and specific tissue activation e.g. in order to enhance angiogenesis (possible ligands could be: specific growth factors (vEGF, bFGF, Epo, etc.), aptamers and peptides with specific "cell recognition" (RGD) sequences (Integrin-ligands, etc.) amongst others);
- Coating the TEMS-tissue interface with pharmacologically active substances either directly or an a biocompatible carrier for targeted drug application to the site of dysfunctional myocardium (possible pharmaceutical substances could be: COX-inhibitors, RAS-modulators, anti-fibrotic substances, etc.);
- Culturing autologous cells with regenerative or contractile potential on the TEMS-tissue interface either directly or an a biocompatible carrier for targeted cell application to the site of dysfunctional myocardium (possible cell sources: tissue derived (heart-specific) progenitor- and/or stemcells, circulating endothelilal progenitor cells, autologous (adult) stemcells, bone marrow derived mesenchyme stem cells, allogene stemcells (such as e.g. USSCs) amongst others); and
- Coating the epicardial site of the TEMS with biodegradable materials such as different hydrogels in order to reduce possible inflammation or increase biomechanical properties (Possible hydrogels: methacrylated synthetic or biological materials such as PEG, GelMA, HA, etc.).

### Vascularized TEMS:

After the decellularization process, the vascular system of the donor heart remains intact in terms of patency and ECM-integrity and can be successfully re-endothelialized with human endothelial cells (Aubin et al., TISSUE ENG, 2013):

### Standardized native derived coronary artery tissue-flap model:

The standardized coronary artery tissue-flap model was created by microsurgical dissection of the decellularized whole hearts into two tissue flaps adherent to the ascending aorta and encasing the LCA and RCA, respectively. The transluminescence of the decellularized tissue flaps allowed for live imaging via a common inverse field microscope (DM IL LED; Leica) or confocal microscope (LSM 700; Zeiss). Selective catheterization of the coronary ostia via a 28G catheter (Braun) allowed for standardized perfusion of the tissue flaps via the respective de-endothelialized or repopulated main coronary arteries and subsequent vessel system with no perfusate loss, either manually with a syringe or with a syringe pump ensuring a constant flow rate. Coronary artery tissue-flap models with an in vitro repopulated vessel system were created in a two-step procedure. In the first step, the coronary artery vessel system of the whole hearts was cell seeded through coronary perfusion via retrograde aortic perfusion with a cell density of 5x10⁵ cells·mL⁻¹ and incubated in the cell-specific standard culture medium in a standard cell culture incubator (Forma Scientific) at 5% CO₂ atmosphere and 37C. After 24 h, in a second step, repopulated whole hearts were dissected into the above-described coronary artery tissue-flap model under sterile conditions and further incubated for 5 days with a medium renewal via manual coronary perfusion every 12 h. For controlled co-culture with primary cardiac cells, coronary artery tissue flaps were surface seeded with cells at a density of 2x10⁵ cells·mL⁻¹ immediately after dissection and cultivated for at least an additional 24 h.

Although anastomoszing the TEMS vascular system to the host circulation is not possible in the rodent model because of the anatomic dimensions, this might become an option in a larger model, where even only selective areas of the TEMS congruently with the donor coronary system correspond to one of the major coronary arteries may be selectively connected to the host blood flow via a state of the bypass.

The invention is further described in detail with reference to the figures.

### Brief description of the figures

**Figure 1** shows a line diagram of the weight increase of the examined rats over time (after 0 d, 14 d, 42 d; d = days).
   Examined groups:
   1) - LAD / - TEMS (no ligature / no TEMS = Sham)
   2) + LAD / - TEMS (LAD ligature / no TEMS = Infarct)
   3) - LAD / + TEMS (no ligature / TEMS = Tems native)
   4) + LAD / + TEMS (LAD ligature / TEMS = Tems Infarct)
**Figure 2** shows a line diagram of the percentage fractional shortening (FS) over time (after 0 d, 14 d, 42 d; d = days).
**Figure 3** shows a line diagram of the percentage ejection fraction (EF) over time (after 0 d, 14 d, 42 d; d = days).
**Figure 4** shows a line diagram of the percentage fractional area shortening (FAS) over time (after 0 d, 14 d, 42 d; d = days).
**Figure 5** shows a bar diagram of the relative changes of the cardiac function (EF (left bar), FS (middle bar), FAS (right bar)) for all examined groups 1) - 4).
**Figure 6** shows a micrograph of a section of a rat heart covered by a TEMS.
**Figure 7** shows bar diagrams of relative gene expression levels of genes Nppb (upper diagram) and FGF2 (lower diagram) standardized against a) ACTB and b) Top2B.

### Determination of the interaction between a decellularized ECM scaffold (TEMS) and native/damaged myocardial tissue in a rat model:

LAD ligatures were introduced in rat hearts (ligature of the R. interventricularis **anterior;** the "ligature" consists of a piece of thread tied around the blood vessel to shut it off). After 14 days (and 42 days) echocardiograms were made (FAS<40%) and then TEMSs were implanted. After 4 and 8 weeks the hearts were explanted and examined.

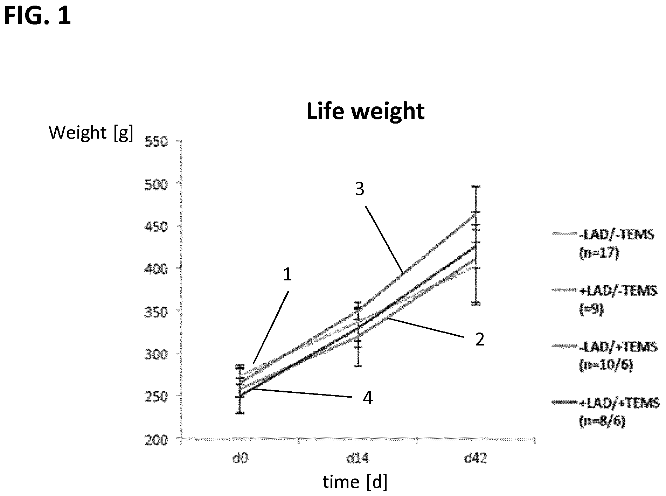

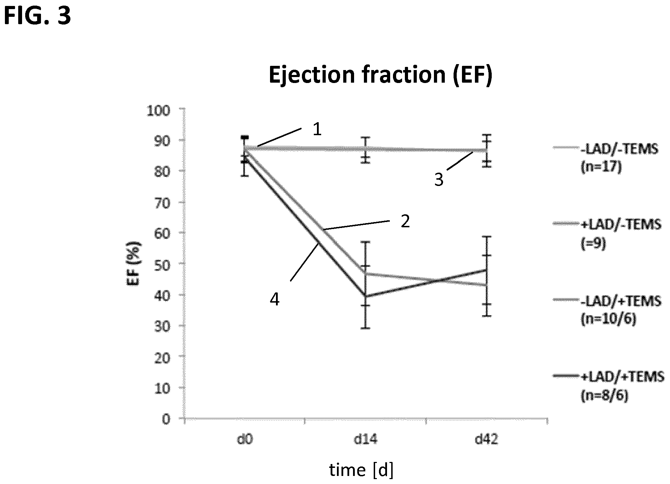

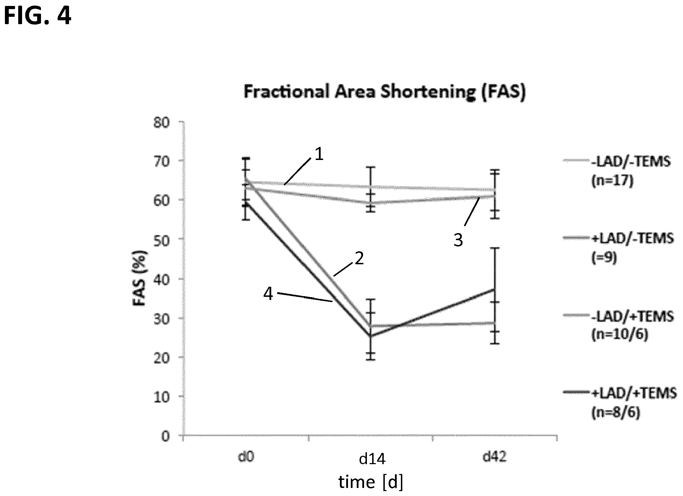

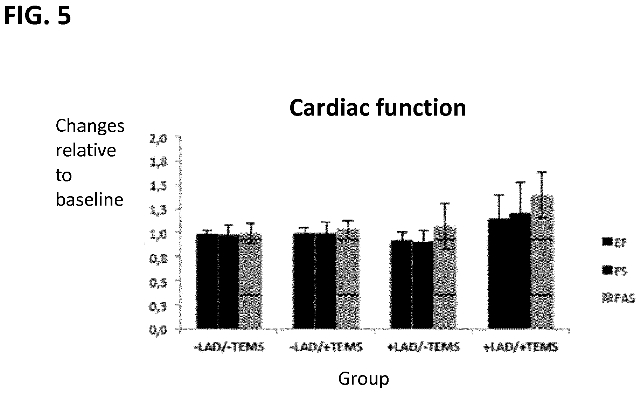

The results of the echocardiographic examination are shown in **Figures 1 to 5****.**

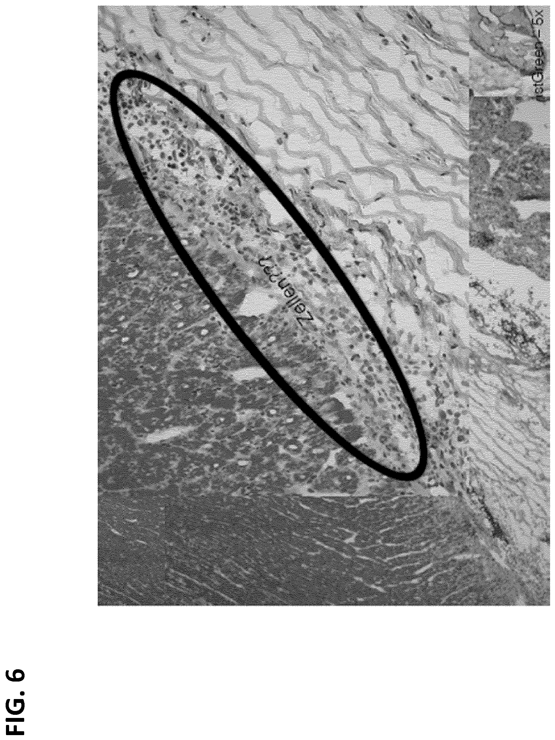

The result of the histologic examination is shown in **Figure 6****.**

Moreover, gene expression in heart muscle cells taken from the anterior or posterior wall of the explanted heart was analyzed by RT-PCR (n=7/group) for all examined groups. Following genes have been analyzed:

| | |
|---|---|
| Housekeeping genes: | Beta-2-microglobuline (cell nucleus enzyme); |
| | Top2b (component of MHC1 complex). |
| Immunmodulation genes: | Interleukin 10 (IL10); |
| | Transforming growth factor, beta receptor II (Tgfbr2); |
| | Tumor necrosis factor alpha (TNFα). |
| Pro angiogenic genes: | Vascular endothelial growth factor A (Vegfa); |
| | Fibroblast growth factor 2 (FGF2); |
| | Placental growth factor (Pgf); |
| | Platelet-derived growth factor beta polypeptide (Pdgfb). |
| Pro survival genes: | Hepatocyte growth factor (hgf); |
| | Stromal cell-derived factor 1/Cxcl12 (Sdf1); |
| | Insulin-like growth factor 1 (Igf1); |
| | V-akt murine thymoma viral oncogene homolog 1/Proteinkinase B α (Akt1). |
| Remodeling genes: | Matrix metalloproteinase 2 (MMP2); |
| | Matrix metalloproteinase 9 (MMP9); |
| | Tissue inhibitor of metalloproteinase 1 (Timp1). |
| Infarct genes: | Natriuretic peptide A/ANP (Nppa); |
| | Natriuretic peptide B/BNP (Nppb). |

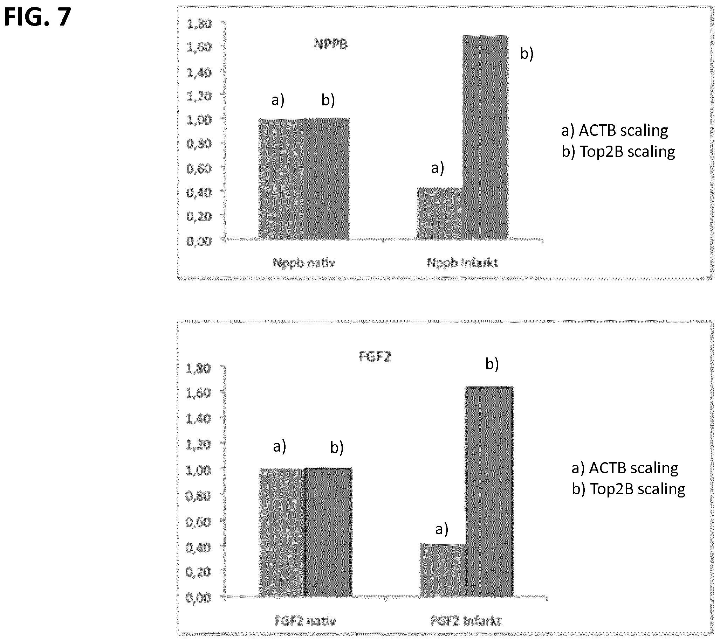

An analysis of the expression of two different genes (Nppb and FGF2) is shown in **Figure 7****.**

Comparison of groups by RT-PCR:
1) Sham vs. Infarct (Anterior wall)
2) Sham vs. Infarct (Posterior wall)
3) Infarct vs. Infarct Tems
4) Tems native vs. Tems Infarct (Tems anterior wall)

1) As shown in **Table 1**, the LAD ligature has a significant effect on gene expression in heart tissue of the anterior wall. In particular, in Infarct samples an increase of the expression of genes IL10, TNFα, TGfbr2, hgf, SDF1, Igf1, MMP2, Timp1, Nppa, and Nppb can be observed. The pro angiogenic factors show both increasing expression (FGF2, Pgf) and decreasing expression (Vegfa, Pdgfb).

2) As shown in **Table 2** (control), there is no difference between the Infarct samples from the posterior wall and the Sham samples from the posterior wall. That is, in contrast to the samples from the anterior wall, no significant differences in gene expression occur between the Sham samples and the Infarct samples. Accordingly, the results according to Table 1 (anterior wall) seem to be valid.

3) As shown in Table 3, treatment of the heart with the TEMS results in a repression of TNFα, Tgfbr2, Nppa, Nppb, Vegfa, and Pdgfb (immunmodulators, infarct parameters and angiogenesis factors). In contrast, enhanced gene expression can be observed for pro survival factors (Hgf, Igf1, [SDF1]).

4) As shown in Table 4, that RNA is detected in the TEMS, i.e. cells are migrated from the heart tissue into the TEMS. No differences in gene expression can be observed between the TEMS matrix from a native heart and the TEMS matrix from an infarct heart.

**Table 1: Results of gene expression analysis for group 1) (Sham vs. Infarct, VW = Anterior wall)**

| | | VW Sham MW±SD | VW Inf. MW±SD | Signifikanz | VW Sham MW±SD | VW Inf. MW±SD | Signifikanz | Tendenz |
|---|---|---|---|---|---|---|---|---|
| Immunmod. | IL10 | 0,63±0,82 | 0,93±0,73 | n.s. | 0,51±0,66 | 0,57±0,39 | n.s. | ⇑ ⇒ |
| | TNFa | 1,49±0,72 | 2,01±0,51 | n.s | 1,71±0,83 | 1,98±0,54 | n.s. | ⇑ |
| | TGFBR2 | 1,49±0,21 | 2,07±0,58 | p<0,001 | 1,78±2,72 | 1,53±0,86 | n.s. | ⇑ |
| | | | | | | | | |
| Pro-angio | Vegfa | 0,87±0,21 | 0,4±0,19 | p<0,001 | 0,77±0,44 | 0,27±0,12 | p<0,05 | ⇓ |
| | FGF2 | 1,34±0,23 | 3,25±0,96 | p<0,001 | 1,66±0,9 | 3,22±1,01 | p<0,05 | ⇑ |
| | Pgf | 1,27±0,31 | 2,42±0,7 | p<0,01 | 1,07±0,53 | 1,62±0,45 | n.s. | ⇑ |
| | Pdgfb | 0,81±0,23 | 0,68±0,22 | n.s. | 0,71±0,37 | 0,46±0,15 | n.s. | ⇓ |
| | | | | | | | | |
| Pro-survival | Hgf | 0,83±0,16 | 3,54±1,06 | p<0,001 | 0,7±0,32 | 2,38±0,7 | p<0,001 | ⇑ |
| | SDF1 | 2,2±1,45 | 7,32±4,17 | p<0,01 | 2,71±1,69 | 7,19±4,6 | p<0,05 | ⇑ |
| | IGF1 | 0,71±0,31 | 8,83±6,45 | p<0,01 | 2,01±3,73 | 5,54±2,14 | n.s. | ⇑ |
| | Aktl | 0,94±0,16 | 0,73±0,16 | p<0,05 | 2,02±3,05 | 0,54±0,22 | n.s. | ⇓ |
| | | | | | | | | |
| Remodeling | Timp1 | 1,3±0,36 | 10,47±3,49 | p<0,001 | 1,04±1,04 | 6,83±2,83 | p<0,001 | ⇑ |
| | MMP2 | 0,78±0,19 | 4,33±1,79 | p<0,001 | 1,76±2,71 | 3,08±1,53 | n.s. | ⇑ |
| | MMP 9 | 1,41±0,86 | 10,63±11,06 | p<0,05 | 0,96±0,52 | 6,28±6,13 | p<0,05 | ⇑ |
| | | | | | | | | |
| Infarkt | Nppa | 0,48±0,38 | 5,05±0,51 | p<0,001 | 0,31±0,31 | 3,34±1,93 | p<0,01 | ⇑ |
| | Nppb | 1,22±0,66 | 1,94±0,77 | n.s. | 1,38±0,83 | 1,86±0,62 | n.s. | ⇑ |
| | | Top2b | | | B2M | | | |

**Table 2: Results of gene expression analysis for group 2) (Sham vs. Infarct, HW = Posterior wall)**

| | | HW Sham MW±SD | HW Infarkt MW±SD | Signifikanz | HW Sham MW±SD | HW Infarkt MW±SD | Signifikanz | Tendenz |
|---|---|---|---|---|---|---|---|---|
| Immunmod. | IL10 | 0,74±0,97 | 1,05±0,82 | n.s. | 0,41±0,83 | 0,36±0,53 | n.s. | ⇒ |
| | TNFa | 0,85±0,32 | 0,96±0,3 | n.s. | 0,78±0,48 | 0,68±0,21 | n.s. | ⇒ |
| | TGFBR2 | 0,86±0,12 | 1,02±0,19 | n.s. | 0,62±0,36 | 0,52±0,1 | n.s. | ⇑ ⇒ |
| | | | | | | | | |
| Pro-angio | Vegfa | 1,07±0,22 | 0,75±0,32 | n.s. | 0,98±0,29 | 0,62±0,36 | n.s. | ⇓ |
| | FGF2 | 1,33±0,24 | 1,35±0,38 | n.s. | 1,26±0,56 | 0,99±0,46 | n.s. | ⇒ |
| | Pgf | 1,25±0,28 | 1,83±1,03 | n.s. | 1,19±0,49 | 1,31±0,62 | n.s. | ⇑ |
| | Pdgfb | 1.02±0,19 | 0,94±0,19 | n.s. | 0,93±0,24 | 0,74±0,27 | n.s. | ⇒ ⇓ |
| | | | | | | | | |
| Pro-survival | Hgf | 1,15±0,4 | 2,74±0,98 | p<0,001 | 1,02±0,27 | 2,02±0,53 | p<0,001 | ⇑ |
| | SDF1 | 0,96±0,36 | 0,94±0,34 | n.s. | 0,93±0,59 | 0,66±0,24 | n.s. | ⇒ |
| | IGF1 | 1,05±0,28 | 1,6±0,62 | n.s. | 0,75±0,42 | 0,79±0,27 | n.s. | ⇑ ⇒ |
| | Akt1 | 0,88±0,17 | 0,86±0,18 | n.s. | 0,63±0,33 | 0,45±0,15 | n.s. | ⇒ ⇓ |
| | | | | | | | | |
| Remodeling | Timp1 | 1,33±0,46 | 2,74±1,65 | p<0,05 | 1,22±0,64 | 1,93±1,45 | n.s. | ⇑ |
| | MMP2 | 0,87±0,17 | 1,23±0,43 | n.s. | 0,66±0,46 | 0,62±0,16 | n.s. | ⇑ ⇒ |
| | MMP9 | 3,42±2,0 | 10,6±13,6 | n.s. | 5,05±3,78 | 17,76±17,64 | n.s. | ⇑ |
| | | | | | | | | |
| Infarkt | Nppa | 0,42±0,28 | 3,62±3,29 | p<0,05 | 0,41±0,31 | 2,65±2,83 | n.s. | ⇑ |
| | Nppb | 1,04±0,17 | 2,6±0,69 | p<0,001 | 0,98±0,36 | 1,79±0,27 | p<0,001 | ⇑ |
| | | Top2b | | | B2M | | | |

**Table 3: Results of gene expression analysis for group 3) (Infarct vs. Infarct Tems, VW = Anterior wall)**

| | | VW Inf. MW±SD | VW Tems Inf. MW±SD | Signifikanz | VW Inf. MW±SD | VW Tems Infarkt MW±SD | Signifikanz | Tendenz |
|---|---|---|---|---|---|---|---|---|
| Immunmod. | IL10 | 1,44±0,67 | 4,06±3,94 | n.s. | 1,44±0,82 | 2,46±1,13 | n.s. | ⇑ |
| | TNF a | 1,4±0,37 | 1,3±0,48 | n.s. | 1,36±0,47 | 0,96±0,41 | n.s. | ⇓ |
| | TGFBR2 | 0,92±0,16 | 0,69±0,16 | p<0,05 | 0,88±0,16 | 0,67±0,18 | p<0,05 | ⇓ |
| | | | | | | | | |
| Pro-angio | Vegfa | 0,99±0,33 | 0,76±0,44 | n.s. | 0,89±0,37 | 0,79±0,53 | n.s. | ⇓ |
| | FGF2 | 0,77±0,21 | 0,82±0,2 | n.s. | 0,77±0,33 | 0,68±0,38 | n.s. | ⇒ |
| | Pgf | 1,27±0,38 | 1,73±0,79 | n.s. | 1,1±0,28 | 1,5±0,44 | n.s. | ⇑ |
| | Pdgfb | 1,07±0,16 | 0,78±0,13 | p<0,01 | 0,94±0,17 | 0,75±0,27 | n.s. | ⇓ |
| | | | | | | | | |
| Pro-survival | Hgf | 1,36±0,53 | 1,61±0,54 | n.s. | 1,16±0,33 | 1,45±0,5 | n.s. | ⇑ |
| | SDF1 | 1,12±0,59 | 1,52±1,2 | n.s. | 1,19±0,91 | 0,96±0,49 | n.s. | ⇑ ⇒ |
| | IGF1 | 1,03±0,48 | 1,53±0,68 | n.s. | 0,98±0,34 | 1,41±0,53 | n.s. | ⇑ |
| | Akt1 | 0,95±0,44 | 0,74±0,27 | n.s. | 0,89±0,29 | 0,77±0,33 | n.s. | ⇓ |
| | | | | | | | | |
| Remodeling | Timp1 | 0,86±0,34 | 1,07±0,46 | n.s. | 0,81±0,25 | 0,83±0,58 | n.s. | ⇑ ⇒ |
| | MMP2 | 1,01±0,33 | 0,96±0,36 | n.s. | 0,97±0,28 | 0,93±0,38 | n.s. | ⇒ |
| | MMP 9 | 2,61±2,88 | 7,36±9,96 | n.s. | 2,57±3,07 | 6,87±12,7 | n.s. | ⇑ |
| | | | | | | | | |
| Infarkt | Nppa | 0,73±,46 | 0,48±0,34 | n.s. | 0,71±0,45 | 0,37±0,29 | n.s. | ⇓ |
| | Nppb | 0,9±0,16 | 0,67±0,43 | n.s. | 0,9±0,33 | 0,5±0,27 | p<0,05 | ⇓ |
| | | Top2b | | | | | | |

**Table 4: Results of gene expression analysis for group 4) (Tems native vs. Tems Infarct (VW = anterior wall), determination of gene expression in cells which are migrated into the TEMS (TEMS was free of cells before implantation)**

| | | Tems VW Tems nativ MW±SD | Tems VW Tems Inf. MW±SD | Signifikanz | Tems VW Tems nativ MW±SD | Tems VW Tems Inf. MW±SD | Signifikanz | Tendenz |
|---|---|---|---|---|---|---|---|---|
| Immunmod. | IL10 | 2,33±1,42 | 2,52±1,28 | n.s. | 1,61±1,17 | 1,47±1,25 | n.s. | ⇑ ⇓ |
| | TNF a | 1,22±0,55 | 1,16±0,54 | n.s. | 1,15±0,66 | 0,91±0,39 | n.s. | ⇒ ⇓ |
| | TGFBR2 | 0,96±0,39 | 0,92±0,16 | n.s. | 1,55±1,21 | 1,52±0,75 | n.s. | ⇒ |
| | | | | | | | | |
| Pro-angio | Vegfa | 1,06±0,53 | 0,71±0,21 | n.s. | 0,95±0,7 | 0,68±0,47 | n.s. | ⇓ |
| | FGF2 | 0,73±0,31 | 1,1±0,62 | n.s. | 0,95±0,5 | 0,68±1,32 | n.s. | ⇑ ⇓ |
| | Pgf | 0,96±0,29 | 1,35±0,39 | n.s. | 0,88±0,45 | 1,24±0,81 | n.s. | ⇑ |
| | Pdgfb | 1,07±0,28 | 0,74±0,32 | n.s. | 0,91±0,27 | 0,7±0,46 | n.s. | ⇓ |
| | | | | | | | | |
| Pro-survival | Hgf | 1,04±0,3 | 1,1±0,3 | n.s. | 0,92±0,46 | 1±0,6 | n.s. | ⇒ |
| | SDF1 | 3,04±2,44 | 3,97±3,35 | n.s. | 2,76±2,49 | 2,52±1,25 | n.s. | ⇑ ⇓ |
| | IGF1 | 1,06±0,4 | 1,12±0,31 | n.s. | 1,6±0,97 | 1,51±0,9 | n.s. | ⇒ |
| | Akt1 | 0,98±0,16 | 1,03±0,12 | n.s. | 1,48±0,84 | 1,39±0,76 | n.s. | ⇒ |
| | | | | | | | | |
| Remodeling | Timp1 | 0,66±0,25 | 1,18±0,7 | n.s. | 0,48±0,29 | 0,91±0,8 | n.s. | ⇑ |
| | MMP2 | 0,75±0,3 | 0,93±0,35 | n.s. | 1,2±0,93 | 1,29±1,02 | n.s. | ⇑ |
| | MMP 9 | 2,94±2,24 | 1,2±0,62 | n.s. | 1,86±1,56 | 0,83±0,6 | n.s. | ⇓ |
| | | | | | | | | |
| Infarkt | Nppa | 29,2±40,37 | 3,17±3,59 | n.s. | 19,54±28,33 | 2,57±3,86 | n.s. | ⇓ |
| | Nppb | 30,05±37,8 | 2,75±2,62 | n.s. | 30,92±45,9 | 2,74±3,02 | n.s. | ⇓ |
| | | Top2b | | | | | | |

As a result, the experiments show that cardinal gene expression is altered after a LAD ligature, particularly gene expression is increased in respect of remodeling, immunmodulation, pro survival and infarct factors. Treatment of a damaged heart with the TEMS results in decreasing expression of immunmodulation and infarct factors and increasing expression of pro survival factors.

In summary, the invention provides a customized tissue engineered myocardial sleeve (TEMS) based on a bioactive extracellular matrix (ECM) scaffold directly derived from native cardiac tissue, to circumferentially encase the diseased heart in order to promote regeneration of the dysfunctional myocardium and improve function of the diseased heart.

By providing structural and mechanical support, diastolic function is improved through prevention of ventricular dilatation (passive cardiomyoplasty). Structure and function of diseased myocardium can be reestablished by promoting endogenous tissue regeneration through host cell-recruitment, - migration, and -differentiation (bioactive cardiomyoplasty).

Further, exogenously induced tissue regeneration is promoted by customized TEMS (cTEMS) delivering biological clues in form of physiologically or pharmacologically active substances, e.g. for targeted host-cell recruitment or enhancement of angiogenesis, and/or donor cells with regenerative potential directly to the site of dysfunctional myocardium (targeted cardiomyoplasty).

Moreover, systolic function is improved by recruitment, differentiation and/or targeted application of contractile cells into the sites of diseased myocardium and adjacent TEMS (active cardiomyoplasty)

### 5. Literature

Gummert JF, Rahmel A, Bossert T, Mohr FW.Socks for the dilated heart. Does passive cardiomyoplasty have a role in long-term care for heart failure patients? Z Kardiol. 2004 Nov;93(11):849-54.
Grothues F, Huth C, Klein HU. MRI of a novel passive cardiomyoplasty device: the Paracor ventricular support system. Heart. 2006 Sep;92(9):1224.
Mann DL, Kubo SH, Sabbah HN, Starling RC, Jessup M, Oh JK, Acker MA. Beneficial effects of the CorCap cardiac support device: Five-year results from the Acorn Trial. J Thorac Cardiovasc Surg. 2011 Jul 13
Akhyari P, Aubin H, Gwanmesia P, Barth M, Hoffmann S, Huelsmann J, Preuss K, Lichtenberg A. The quest for an optimized protocol for whole-heart decellularization: a comparison of three popular and a novel decellularization technique and their diverse effects on crucial extracellular matrix qualities. Tissue Eng Part C Methods. 2011 Sep;17(9):915-26.
Crapo PM, Gilbert TW, Badylak SF. An overview of tissue and whole organ decellularization processes. Biomaterials. 2011 Apr;32(12):3233-43.
Aubin H, Kranz A, Hülsmann J, Lichtenberg A, Akhyari P. Decellularized whole heart for bioartificial heart. Methods Mol Biol. 2013;1036:163-78.
Assmann A, Delfs C, Munakata H, Schiffer F, Horstkötter K, Huynh K, Barth M, Stoldt VR, Kamiya H, Boeken U, Lichtenberg A, Akhyari P. Acceleration of autologous in vivo recellularization of decellularized aortic conduits by fibronectin surface coating. Biomaterials. 2013 Aug;34(25):6015-26.
Camci-Unal G, Aubin H, Ahari AF, Bae H, Nichol JW, Khademhosseini A. Surface-modified hyaluronic acid hydrogels to capture endothelial progenitor cells. Soft Matter. 2010 Oct 21;6(20):5120-5126.
Aubin H, Kranz A, Huelsmann J, Pinto A, Barth M, Fomin A, Lichtenberg A, Akhyari P. A novel native derived coronary artery tissue-flap model. Tissue Eng Part C Methods. 2013 Apr 30.

## Claims

1. Method for producing a support device, said method comprising:
a) providing a whole heart comprising an extracellular matrix and cells attached thereto;
b) removing all cells from the extracellular matrix so as to produce a decellularized extracellular matrix scaffold; and
c) processing the extracellular matrix scaffold so as to produce a sleeve-like support device which is designed to circumferentially encase a living heart, wherein the extracellular matrix scaffold is processed by removing at least one part of at least one of the heart structures selected from the group consisting of the heart base, the apex, and the septal wall.

2. Method according to claim 1, wherein the extracellular matrix scaffold is processed in step c) by treating the surface of the extracellular matrix scaffold.

3. Method according to claim 2, wherein the treatment is performed by laser manipulation techniques.

4. Method according to any one of claims 1 to 3, wherein the endocardial layer on at least a part of the surface of the extracellular matrix scaffold is at least in part destroyed and/or removed.

5. Method according to any one of claims 1 to 4, wherein the extracellular matrix scaffold is additionally customized by coating the surface of the extracellular matrix scaffold.

6. Method according to claim 5, wherein the surface of the extracellular matrix scaffold is coated with at least one growth factor, aptamer, RGD peptide, pharmacologically active substance, and/or biodegradable material.

7. Method according to any one of claims 1 to 6, wherein the extracellular matrix scaffold is customized by recolonizing the extracellular matrix with living cells.

8. Method according to claim 7, wherein the extracellular matrix scaffold is recolonized with at least one cell selected from the group consisting of tissue derived progenitor or stem cells, circulating endothelial progenitor cells, autologous or allogene stem cells, and bone marrow derived mesenchyme stem cells.

9. Support device comprising a decellularized extracellular matrix scaffold of a heart, said extracellular matrix scaffold being designed like a sleeve to circumferentially encase a living heart, wherein the extracellular matrix scaffold is devoid of at least one part of at least one of the heart structures selected from the group consisting of the heart base, the apex, and the septal wall.

10. Support device according to claim 9, wherein the extracellular matrix scaffold comprises the free walls of the left and right ventricles of the heart.

11. Support device according to claim 9 or 10, wherein at least a part of the surface of the extracellular matrix scaffold is devoid of an endocardial layer.

12. Support device according to any one of claims 9 to 11, wherein the extracellular matrix scaffold is coated with at least one growth factor, aptamer, RGD peptide, pharmacologically active substance, and/or biodegradable material.

13. Support device according to any one of claims 9 to 12, wherein the extracellular matrix scaffold is colonized with living cells.

14. Support device according to claim 13, wherein the extracellular matrix scaffold is colonized with at least one cell selected from the group consisting of tissue derived progenitor or stem cells, circulating endothelial progenitor cells, autologous or allogene stem cells, and bone marrow derived mesenchyme stem cells.

## Patentansprüche

1. Verfahren zur Herstellung einer Stützvorrichtung, welches umfasst:
a) Bereitstellen eines ganzen Herzens, welches eine extrazelluläre Matrix und daran anhaftende Zellen umfasst;
b) Entfernen aller Zellen von der extrazellulären Matrix, um ein dezellularisiertes Gerüst der extrazellulären Matrix herzustellen; und
c) Aufbereiten des Gerüsts der extrazellulären Matrix, um eine manschettenartige Stützvorrichtung herzustellen, die zum umfänglichen Ummanteln eines lebenden Herzens ausgebildet ist, wobei das Gerüst der extrazellulären Matrix durch Entfernen mindestens eines Teils mindestens einer der Herzstrukturen, die aus der Gruppe bestehend aus der Herzbasis, dem Apex und der Scheidewand ausgewählt ist, aufbereitet wird.

2. Verfahren nach Anspruch 1, wobei das Gerüst der extrazellulären Matrix in Schritt c) durch Behandlung der Oberfläche des Gerüst der extrazellulären Matrix aufbereitet wird.

3. Verfahren nach Anspruch 2, wobei die Behandlung durch Laserbearbeitungstechniken durchgeführt wird.

4. Verfahren nach einem der Ansprüche 1 bis 3, wobei die Endokardschicht auf mindestens einem Teil der Oberfläche des Gerüsts der extrazellulären Matrix zumindest teilweise zerstört und/oder entfernt wird.

5. Verfahren nach einem der Ansprüche 1 bis 4, wobei das Gerüst der extrazellulären Matrix zusätzlich durch Beschichtung der Oberfläche des Gerüsts der extrazellulären Matrix angepasst wird.

6. Verfahren nach Anspruch 5, wobei die Oberfläche des Gerüsts der extrazellulären Matrix mit mindestens einem Wachstumsfaktor, Aptamer, RGD-Peptid, einer pharmakologisch aktiven Substanz und/oder einem biologisch abbaubaren Material beschichtet wird.

7. Verfahren nach einem der Ansprüche 1 bis 6, wobei das Gerüst der extrazellulären Matrix durch Wiederbesiedelung der extrazellulären Matrix mit lebenden Zellen angepasst wird.

8. Verfahren nach Anspruch 7, wobei das Gerüst der extrazellulären Matrix mit mindestens einer Zelle, die aus der Gruppe bestehend aus von Gewebe abstammenden Vorläufer- oder Stammzellen, zirkulierenden endothelialen Vorläuferzellen, autologen oder allogenen Stammzellen und von Knochenmark abstammenden mesenchymalen Stammzellen ausgewählt ist, wiederbesiedelt wird.

9. Stützvorrichtung, die ein dezellularisiertes Gerüst einer extrazellulären Matrix eines Herzens umfasst, wobei dieses Gerüst der extrazellulären Matrix wie eine Manschette zum umfänglichen Ummanteln eines lebenden Herzens ausgebildet ist, wobei das Gerüst der extrazellulären Matrix frei von mindestens einem Teil mindestens einer der Herzstrukturen ist, die aus der Gruppe bestehend aus der Herzbasis, dem Apex und der Scheidewand ausgewählt ist.

10. Stützvorrichtung nach Anspruch 9, wobei das Gerüst der extrazellulären Matrix the freien Wände der linken und rechten Herzkammern umfasst.

11. Stützvorrichtung nach Anspruch 9 oder 10, wobei mindestens ein Teil der Oberfläche des Gerüsts der extrazellulären Matrix frei von einer Endokardschicht ist.

12. Stützvorrichtung nach einem der Ansprüche 9 bis 11, wobei das Gerüst der extrazellulären Matrix mit mindestens einem Wachstumsfaktor, Aptamer, RGD-Peptid, einer pharmakologisch aktiven Substanz und/oder einem biologisch abbaubaren Material beschichtet ist.

13. Stützvorrichtung nach einem der Ansprüche 9 bis 12, wobei das Gerüst der extrazellulären Matrix mit lebenden Zellen besiedelt ist.

14. Stützvorrichtung nach Anspruch 13, wobei das Gerüst der extrazellulären Matrix mit mindestens einer Zelle, die aus der Gruppe bestehend aus von Gewebe abstammenden Vorläufer- oder Stammzellen, zirkulierenden endothelialen Vorläuferzellen, autologen oder allogenen Stammzellen und von Knochenmark abstammenden mesenchymalen Stammzellen ausgewählt ist, besiedelt ist.

## Revendications

1. Procédé de production d'un dispositif de soutien, ledit procédé comprenant :
a) l'apport d'un cœur complet comprenant une matrice extracellulaire et des cellules attachées à celle-ci ;
b) le retrait de toutes les cellules de la matrice extracellulaire de manière à produire un échafaudage matriciel extracellulaire décellullarisé ; et
c) le traitement de l'échafaudage matriciel extracellulaire de manière à produire un dispositif de soutien en forme de manche qui est conçu pour entourer de manière circonférentielle un cœur vivant, dans lequel l'échafaudage matriciel extracellulaire est traité par l'élimination d'au moins une partie d'au moins une des structures cardiaques choisies dans le groupe constitué de la base du cœur, de l'apex, et de la paroi septale.

2. Procédé selon la revendication 1, dans lequel l'échafaudage matriciel extracellulaire est traité dans l'étape c) par traitement de la surface de l'échafaudage matriciel extracellulaire.

3. Procédé selon la revendication 2, dans lequel le traitement est effectué au moyen de techniques de manipulation de laser.

4. Procédé selon l'une quelconque des revendications 1 à 3, dans lequel la couche endocardiale sur au moins une partie de la surface de l'échafaudage matriciel extracellulaire est au moins en partie détruite et/ou éliminée.

5. Procédé selon l'une quelconque des revendications 1 à 4, dans lequel l'échafaudage matriciel extracellulaire est en outre adapté sur mesure au moyen du revêtement de la surface de l'échafaudage matriciel extracellulaire.

6. Procédé selon la revendication 5, dans lequel la surface de l'échafaudage matriciel extracellulaire est revêtue avec au moins un facteur de croissance, un aptamère, un peptide RGD, une substance pharmacologiquement active, et/ou un matériau biodégradable.

7. Procédé selon l'une quelconque des revendications 1 à 6, dans lequel l'échafaudage matriciel extracellulaire est adapté sur mesure au moyen de la recolonisation de la matrice extracellulaire par des cellules vivantes.

8. Procédé selon la revendication 7, dans lequel l'échafaudage matriciel extracellulaire est recolonisé par au moins une cellule choisie dans le groupe constitué de cellules progénitrices ou souches dérivées de tissus, de cellules progénitrices endothéliales en circulation, de cellules souches autologues ou allogènes, et de cellules souches du mésenchyme dérivées de la moelle osseuse.

9. Dispositif de soutien comprenant un échafaudage matriciel extracellulaire décellullarisé d'un cœur, ledit échafaudage matriciel extracellulaire étant conçu comme une manche afin d'entourer de manière circonférentielle un cœur vivant, dans lequel l'échafaudage matriciel extracellulaire est exempt d'au moins une partie d'au moins une des structures cardiaques choisies dans le groupe constitué de la base du cœur, de l'apex, et de la paroi septale.

10. Dispositif de soutien selon la revendication 9, dans lequel l'échafaudage matriciel extracellulaire comprend les parois libres des ventricules gauche et droit du cœur.

11. Dispositif de soutien selon la revendication 9 ou la revendication 10, dans lequel au moins une partie de la surface de l'échafaudage matriciel extracellulaire est exempte d'une couche endocardiale.

12. Dispositif de soutien selon l'une quelconque des revendications 9 à 11, dans lequel l'échafaudage matriciel extracellulaire est revêtu avec au moins un facteur de croissance, un aptamère, un peptide RGD, une substance pharmacologiquement active, et/ou un matériau biodégradable.

13. Dispositif de soutien selon l'une quelconque des revendications 9 à 12, dans lequel l'échafaudage matriciel extracellulaire est colonisé par des cellules vivantes.

14. Dispositif de soutien selon la revendication 13, dans lequel l'échafaudage matriciel extracellulaire est colonisé par au moins une cellule choisie dans le groupe constitué de cellules progénitrices ou souches dérivées de tissus, de cellules progénitrices endothéliales en circulation, de cellules souches autologues ou allogènes, et de cellules souches du mésenchyme dérivées de la moelle osseuse.
